# EUROPEAN PATENT APPLICATION

(11) **EP 4 556 012 A1**
(43) Date of publication of application: **21.05.2025**
(21) Application number: 23839815.0
(22) Date of filing: 14.06.2023
(51) Int. Cl.: A61K 35/28, A61P 35/00, A23L 33/10, C12N 5/0775

(54) **ANTICANCER COMPOSITION COMPRISING STEM CELL-DERIVED EXOSOMES AND PREPARATION METHOD THEREFOR**

(30) Priority: 11.07.2022 KR 20220084939
(71) Applicant: Brexogen Inc., Seoul 05855 (KR)
(72) Inventor: KIM, Sue, Seoul 05855 (KR); KIM, Jimin, Seoul 05837 (KR); JUNG, Minyoung, Seoul 08722 (KR)
(74) Representative: Plougmann Vingtoft a/s
(86) International application number: PCT/KR2023/008222
(87) International publication number: WO 2024/014721

(57) **Abstract**

The present invention relates to an anticancer composition comprising stem cell-derived exosomes and a preparation method therefor, the composition, according to the present invention, having an excellent anticancer effect and thus being usable as a cancer therapeutic agent independently or along with existing anticancer drugs.

## Description

### Technical Field

The present disclosure relates to a composition including stem cell-derived exosomes and a method for preparing same. More particularly, the present disclosure relates to a composition comprising exosomes isolated from mesenchymal stem cells or a culture thereof, which exhibit excellent anti-cancer effects.

### Background Art

Extracellular vesicles are vesicles composed of a spherical lipid bilayer, with a size of 30 to 1,000 nm, including microvesicles and exosomes.

The lipid bilayer of exosomes has a phospholipid bilayer structure similar to that of the originating (donor) cells. Exosomes are known to perform functional roles such as cell-to-cell communication and mediation of cellular immunity, acting as assemblies of substances secreted by cells into the extracellular space.

Exosomes contain cell-specific components that reflect the unique biological functions of the originating cells, and include phospholipids, mRNA, miRNA, as well as various soluble proteins, peripheral proteins, and transmembrane proteins.

Such exosomes are secreted from all animal cells, including mast cells, lymphocytes, astrocytes, platelets, neurons, endothelial cells, and epithelial cells, and are found in various body fluids such as blood, urine, mucus, saliva, bile, ascites, and cerebrospinal fluid. Exosomes can also pass through the blood-brain barrier (BBB) and have high selective permeability that allows them to permeate the cell membranes of epithelial and endothelial cells. This property makes exosomes useful in the development of drug delivery systems (DDS) as nanocarriers for specific drugs.

Exosomes and microvesicles secreted from mesenchymal stem cells are involved in cell-to-cell communication and are known to exhibit the regenerative therapeutic efficacy inherent to stem cells.

It is known that after transplantation of stem cells into the body, paracrine factors released from the cells bring about trophic effects without the need for long-term survival of the cells. These factors include low-molecular-weight substances such as growth factors, chemokines, and cytokines, which are secreted via extracellular vesicles like exosomes derived from stem cells. Therefore, exosomes are being utilized to elucidate the characteristics of stem cells and to evaluate their therapeutic efficacy. Furthermore, recent research is actively exploring the therapeutic effects on various diseases using exosomes secreted by mesenchymal stem cells without using the mesenchymal stem cells themselves. Academia and industry anticipate that exosomes could be a new alternative to overcome the limitations of existing stem cell therapies.

### Disclosure of Invention

### Technical Problem

A composition derived from stem cells or a culture thereof, developed by the present inventors, has been confirmed to have remarkably superior anti-cancer effects.

Therefore, the present disclosure aims to provide a composition including exosomes derived from stem cells.

Also, the present disclosure is to provide a method for preparing a composition including exosomes derived from stem cells.

In addition, the present disclosure is to provide a composition including exosomes derived from stem cells for alleviating, inhibiting, preventing, or treating cancer.

Furthermore, the present disclosure is to provide a method for preparing a composition for alleviating, inhibiting, preventing, or treating cancer.

Moreover, the present disclosure is to provide a method for alleviating or treating cancer.

Still more, the present disclosure is to provide a use of the composition including exosomes derived from stem cells for alleviating, inhibiting, preventing, or treating cancer.

### Solution to Problem

The present disclosure relates to an anti-cancer composition including stem cell-derived exosomes and a method for preparing same. The composition according to the present disclosure exhibits excellent effects in the treatment or prevention of cancer.

The present inventors have confirmed that the composition according to the present disclosure has superior anti-cancer effects.

Below, a detailed description will be given of the present disclosure.

An aspect of the present disclosure pertains to a composition comprising exosomes derived from stem cells.

The term "exosome", as used herein, refers to a cell-derived vesicle present in the body fluids of almost all eukaryotic organisms. It means a vesicle with a diameter of about 30-100 nm, which is larger than LDL proteins but much smaller than red blood cells. Exosomes are known to be released from cells when multivesicular bodies fuse with the cell membrane or can be directly released from the cell membrane, performing important and specialized functions such as coagulation and intercellular signal transduction.

The term "stem cell", as used herein, refers to an undifferentiated cell capable of self-renewal and differentiation into two or more different types of cells.

In an embodiment of the present disclosure, the stem cells may be autologous or allogeneic stem cells, may be derived from any type of animal including humans and non-human mammals, may be adult-derived stem cells, or may be embryonic stem cells. For example, the stem cells may be selected from the group consisting of embryonic stem cells, adult stem cells, induced pluripotent stem cells (iPSCs), mesenchymal stem cells derived from induced pluripotent stem cells, BxC stem cells, mesenchymal stem cells derived from induced pluripotent stem cells pretreated with interferon gamma (IFN-γ), and BxC-I17 stem cells, but are not limited thereto.

The term "adult stem cell", as used herein, refers to a cell extracted from umbilical cord blood or adult bone marrow, blood, etc., meaning a cell just before differentiating into specific organ cells, and also refers to undifferentiated cells having the ability to develop into tissues within the body when needed.

In an embodiment of the present disclosure, the adult stem cells may be selected from the group consisting of adult stem cells of human, animal, or animal tissue origin; mesenchymal stem cells derived from human, animal, or animal tissues; and mesenchymal stem cells derived from induced pluripotent stem cells of human, animal, or animal tissue origin, but are not limited thereto.

In the present disclosure, the human, animal, or animal tissues may be selected from the group consisting of umbilical cord, umbilical cord blood, bone marrow, fat, muscle, nerve, skin, amniotic membrane, and placenta, but are not limited thereto.

In the present disclosure, stem cells of various tissue origins from humans or animals may be selected from the group consisting of hematopoietic stem cells, mammary stem cells, intestinal stem cells, vascular endothelial stem cells, neural stem cells, olfactory neural stem cells, and testicular stem cells, but are not limited thereto.

The term "embryonic stem cell", as used herein, refers to cells extracted during the development process of an embryo, meaning those cultured ex vivo by extracting the inner cell mass from a blastocyst-stage embryo just before the fertilized egg implants in the mother's uterus.

Embryonic stem cells refer to cells with self-renewal ability that are pluripotent, or cells capable of differentiating into cells of all tissues of an organism that are totipotent. In a broad sense, this term also encompasses embryoid bodies derived from embryonic stem cells.

In the present disclosure, the stem cells may include embryonic stem cells derived from any source such as humans, monkeys, pigs, horses, cows, sheep, dogs, cats, mice, and rabbits, but with no limitations thereto.

The term "induced pluripotent stem cell (iPSC)", as used herein, refers to cells induced to have pluripotent differentiation potential through artificial reprogramming processes from differentiated cells and can be used interchangeably with "reprogrammed stem cells."

The artificial reprogramming process may be performed by introducing viral-mediated reprogramming factors using viruses such as retroviruses, lentiviruses, and Sendai viruses, or by introducing non-viral-mediated reprogramming factors using non-viral vectors, proteins and cell extracts. It may also include reprogramming processes using stem cell extracts, compounds, and the like.

Induced pluripotent stem cells have almost the same characteristics as embryonic stem cells; specifically, they have similar cell morphology, similar gene and protein expression, possess pluripotency in vitro and in vivo, form teratomas, can form chimeric mice when inserted into mouse blastocysts, and allow for germline transmission of genes.

In an embodiment of the present disclosure, the stem cells may be induced pluripotent stem cell-derived mesenchymal stem cells.

In an embodiment of the present disclosure, the composition may comprise exosomes derived from induced pluripotent stem cell-derived mesenchymal stem cells.

The term "mesenchymal stem cell (MSC)", as used herein, refers to stem cells derived from the mesenchyme. Mesenchymal stem cells can differentiate into one or more cells selected from the group consisting of osteoblasts, chondrocytes, adipocytes, or myocytes. Mesenchymal stem cells can be isolated from all types of adult tissues; for example, they can be isolated from bone marrow adipose tissue, umbilical cord, or peripheral blood. The MSC population can be defined as exhibiting specific phenotypes. The population of mesenchymal stem cells differentiated from induced pluripotent stem cells may exhibit the same phenotypic characteristics as the population of conventional mesenchymal stem cells. The mesenchymal stem cell population can be understood as a stem cell population expressing CD105, CD73, and CD90 markers at 95% or higher and expressing CD45, CD34, and SSEA-4 at 2% or lower.

In an embodiment of the present disclosure, the stem cell may be a BxC stem cell.

In an embodiment of the present disclosure, the composition may comprise exosomes derived from BxC stem cells.

As used herein, the term "BxC stem cell" refers to stem cells prepared by culturing induced pluripotent stem cells (iPSCs), isolating a population of iPSCs that do not express the stage-specific embryonic antigen 4 (SSEA-4P protein, and then further culturing same. BxC stem cells are at a stage just before fully differentiating from iPSCs into mesenchymal stem cells and can acquire the properties of fully differentiated mesenchymal stem cells through additional culturing. Therefore, the phenotype of the BxC stem cell population is not entirely identical to that of the mesenchymal stem cell population but can exhibit a similar phenotype in the range of 96% to 99.9% similarity. For example, iPSC populations express the CD90 protein at 0.3%, mesenchymal stem cell populations express it at 99.7%, and BxC stem cell populations can express it at a rate of 96.9%, which is about 98% of mesenchymal stem cells. Accordingly, BxC stem cells can be defined as stem cells that have been differentiated by 96% to 99.9% into mesenchymal stem cells by further culturing iPSCs that do not express the SSEA-4 protein, without fully differentiating into mesenchymal stem cells after culturing iPSCs. BxC stem cells exhibit superior stemness compared to mesenchymal stem cells differentiated from iPSCs in the same manner and can secrete large amounts of proteins related to functionality. Specifically, when passaged more than nine times, the BxC stem cells of the present disclosure show a proliferation capacity difference of more than tenfold compared to mesenchymal stem cells (MSCs) derived from the same tissue, and no decrease in proliferation capacity is observed even after more than twelve passages. Additionally, BxC stem cells exhibit more than twice the expression level of Ki67, a marker related to cell proliferation ability, compared to general mesenchymal stem cells. Furthermore, BxC stem cells can express at higher levels one or more genes selected from the group consisting of ANKRD1, CPE, NKAIN4, LCP1, CCDC3, MAMDC2, CLSTN2, SFTA1P, EPB41L3, PDE1C, EMILIN2, SULT1C4, TRIM58, DENND2A, CADM4, AIF1L, NTM, SHISA2, RASSF4, and ACKR3, and can express at lower levels one or more genes selected from the group consisting of DHRS3, BMPER, IFI6, PRSS12, RDH10, and KCNE4, compared to mesenchymal stem cells differentiated from iPSCs in the same manner.

The term "stemness", as used herein, refers to pluripotency, that is, the ability to generate all cell types, and self-renewal, that is, the ability to indefinitely produce cells similar to themselves. For example, it includes increasing the proliferation ability of stem cells while maintaining undifferentiated cells in an undifferentiated state, increasing telomerase activity, enhancing the expression of stemness-acting signals, or boosting cell motility activity, and encompasses exhibiting one or more of these characteristics.

In an embodiment of the present disclosure, the stem cell may be a mesenchymal stem cell derived from induced pluripotent stem cells that has been pretreated with interferon gamma (IFN-γ).

In an embodiment of the present disclosure, the composition may comprise exosomes derived from induced pluripotent stem cell-derived mesenchymal stem cells that have been pretreated with interferon gamma (IFN-γ).

The term "pretreatment", as used herein, refers to the process of culturing mesenchymal stem cells in a medium supplemented with a specific substance. For example, pretreatment involves additionally culturing mesenchymal stem cells fully differentiated from induced pluripotent stem cells in a medium containing interferon gamma (IFN-γ).

In the present disclosure, interferon gamma can increase the stemness and proliferation ability of stem cells and can enhance the number of stem cell-derived exosomes as well as the content of proteins and RNA within the exosomes.

In an embodiment of the present disclosure, the stem cell may be a BxC-I17 stem cell.

In an embodiment of the present disclosure, the composition may comprise exosomes derived from BxC-I17 stem cells.

The term "BxC-I17 stem cell", as used herein, refers to induced pluripotent stem cell-derived mesenchymal stem cells that have been cultured (pretreated) in a medium containing interferon gamma. In this regard, the induced pluripotent stem cell-derived mesenchymal stem cells may be stem cells fully differentiated into mesenchymal stem cells by culturing BxC stem cells. That is, within this specification, BxC-I17 stem cells refer to mesenchymal stem cells obtained by fully differentiating BxC stem cells into mesenchymal stem cells and then culturing (pretreated) them in a medium containing interferon gamma. Therefore, BxC-I17 stem cells can be obtained by further culturing BxC stem cells to fully differentiate into mesenchymal stem cells, and then culturing them in a medium containing 0.001 to 1,000 ng of interferon gamma, for example, 10 ng, for 12 to 48 hours. Compared to mesenchymal stem cells that have not been pretreated with any substance, BxC-I17 stem cells exhibit about a 240% increase in cell proliferation rate, an increase of about sixfold or more in exosome production efficiency, and more than a sixfold increase in the amount of exosome-derived proteins.

In an embodiment of the present disclosure, interferon gamma may be used for pretreatment in the medium at concentrations ranging from 0.001 to 1,000 ng, 0.005 to 500 ng, 0.01 to 100 ng, 0.05 to 50 ng, 0.1 to 25 ng, 0.5 to 15 ng, 1 to 10 ng, or 5 to 15 ng, and for example, may be pretreated at 10 ng, but is not limited thereto.

Another aspect of the present disclosure pertains to a pharmaceutical composition comprising exosomes derived from stem cells as an active ingredient.

In the present disclosure, the pharmaceutical composition may comprise exosomes isolated from mesenchymal stem cells derived from induced pluripotent stem cells or a culture thereof as an active ingredient.

In the present disclosure, the pharmaceutical composition may comprise exosomes derived from stem cells pretreated with interferon gamma.

In the present disclosure, the pharmaceutical composition may comprise exosomes isolated from induced pluripotent stem cell-derived mesenchymal stem cells or a culture thereof, which have been pretreated with interferon gamma.

In the present disclosure, the pharmaceutical composition may comprise one or more exosomes selected from the group consisting of exosomes derived from BxC stem cells (BxC-e) and exosomes derived from BxC-I17 stem cells (BxC-I17e).

As used herein, the term "including or comprising as an active ingredient" means including an amount sufficient to achieve the alleviation, inhibition, prevention, or therapeutic activity against a specific disease by the exosomes isolated from stem cells or a culture thereof.

In the present disclosure, the pharmaceutical composition may further comprise a pharmaceutically acceptable carrier, and may include, for example, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, mineral oil, and the like, but is not limited thereto.

In the present disclosure, the pharmaceutical composition may further comprise lubricants, wetting agents, sweeteners, flavoring agents, emulsifiers, suspending agents, preservatives, and the like.

In the present disclosure, the pharmaceutical composition may be administered orally or parenterally, for example, by intravenous injection, subcutaneous injection, intramuscular injection, intraperitoneal injection, topical administration, intranasal administration, intrapulmonary administration, rectal administration, intradural administration, ocular administration, skin administration, or transdermal administration, but with no limitations thereto.

In the present disclosure, the dosage of the pharmaceutical composition may vary depending on factors including formulation method, administration method, age, weight, sex, pathological condition, diet, administration time, administration route, excretion rate, and responsiveness of the patient, and can be determined or prescribed in an effective amount for desired treatment or prevention. For example, the daily dosage of the pharmaceutical composition of the present disclosure may be 0.0001-1000 mg/kg.

In the present disclosure, the pharmaceutical composition can be prepared into unit dosage forms or inserted into multi-dose containers by formulating with pharmaceutically acceptable carriers and/or excipients according to methods that can be easily carried out by those skilled in the art to which the present disclosure pertains. At this time, the formulation may be in the form of a solution, suspension, or emulsion in an oil or aqueous medium, or may be in the form of extracts, powders, suppositories, powders, granules, tablets, or capsules, and may additionally include dispersing agents or stabilizers, but with no limitations thereto.

The dosage of the pharmaceutical composition of the present disclosure may vary depending on the patient's age, weight, sex, administration route, health condition, and disease severity, and may be administered once or several times a day at regular intervals according to the judgment of a doctor or pharmacist. For example, based on the content of the active ingredient, the daily dosage may be 1 to 1000 ug/ml, but this is an exemplary average dose and may be higher or lower depending on individual differences.

In the present disclosure, the pharmaceutical composition may be a pharmaceutical composition for treating, preventing, alleviating, or inhibiting cancer.

The term "treatment of cancer", as used herein, means including all actions that suppress cancer, and specifically may mean actions that regulate the cycle of cancer cells or induce apoptosis of cancer cells, but with no limitations thereto.

In an embodiment of the present disclosure, the cancer may be one or more selected from the group consisting of breast cancer, lung cancer, gastric cancer, liver cancer, blood cancer, bone cancer, pancreatic cancer, skin cancer, head or neck cancer, cutaneous or ocular melanoma, uterine sarcoma, ovarian cancer, rectal cancer, anal cancer, colon cancer, fallopian tube cancer, endometrial cancer, cervical cancer, small intestine cancer, endocrine cancer, thyroid cancer, parathyroid cancer, kidney cancer, soft tissue tumors, urethral cancer, prostate cancer, bronchial cancer, glioblastoma, or bone marrow cancer, but is not limited thereto.

In an embodiment of the present disclosure, the cancer may be one or more selected from the group consisting of liver cancer, pancreatic cancer, glioblastoma, colon cancer, breast cancer, prostate cancer, and blood cancer, but is not limited thereto.

Another aspect of the present disclosure pertains to a food composition comprising stem cell-derived exosomes as an active ingredient.

Since the food composition according to the present disclosure comprises the same stem cell-derived exosomes as in the above-described pharmaceutical composition, the common content therebetween is omitted to avoid undue complexity in this specification.

The food composition according to the present disclosure may include ingredients commonly added during food manufacturing, such as proteins, carbohydrates, fats, nutrients, seasonings, and flavorings, but with no limitations thereto.

Carbohydrates that may be included in the food composition according to the present disclosure may include monosaccharides like glucose and fructose; disaccharides like maltose, sucrose, and oligosaccharides; polysaccharides like dextrin and cyclodextrin; and sugar alcohols like xylitol, sorbitol, and erythritol, but are not limited thereto.

Flavorings that may be included in the food composition according to the present disclosure may include natural flavorings such as thaumatin and stevia extracts, and synthetic flavorings such as saccharin and aspartame, but are not limited thereto.

In the present disclosure, the food composition may be a food composition for alleviating, inhibiting, or improving cancer.

Another aspect of the present disclosure pertains to a cell therapeutic agent comprising induced pluripotent stem cell-derived mesenchymal stem cells.

The term "cell therapeutic agent", as used herein, refers to a pharmaceutical product used for treatment, diagnosis, or prevention purposes through a series of actions such as proliferating or selecting living autologous, allogenic, or xenogenic cells ex vivo, or altering the biological characteristics of cells by other methods, in order to restore the functions of cells and tissues.

In an embodiment of the present disclosure, the cell therapeutic agent may be a stem cell therapeutic agent.

In an embodiment of the present disclosure, the induced pluripotent stem cell-derived mesenchymal stem cells may comprise BxC-I17 stem cells.

In the present disclosure, the cell therapeutic agent may be a cell therapeutic agent for alleviating, inhibiting, preventing, or treating cancer.

Another aspect of the present disclosure pertains to a method for preparing a composition comprising exosomes isolated from stem cells or a culture thereof, the method comprising the following step:

a separation step of isolating exosomes from stem cells or a culture thereof.

In the present disclosure, the method may be a method for preparing a composition comprising exosomes isolated from induced pluripotent stem cell-derived mesenchymal stem cells or a culture thereof.

In an embodiment of the present disclosure, the separation step may comprise isolating exosomes from induced pluripotent stem cell-derived mesenchymal stem cells or a culture thereof.

In an embodiment of the present disclosure, the stem cells may be autologous or allogeneic stem cells, may be derived from any type of animal including humans and non-human mammals, may be adult-derived stem cells, or may be embryo-derived stem cells. For example, the stem cells may be embryonic stem cells, adult stem cells, induced pluripotent stem cells (iPSCs), mesenchymal stem cells derived from induced pluripotent stem cells, BxC stem cells, or BxC-I17 stem cells, but are not limited thereto.

In the separation step, after centrifuging the culture medium of the stem cells at 200-400xg for 5 to 20 minutes to remove remaining cells and cell debris, the supernatant is collected and centrifuged at 9,000-12,000xg for 60 to 80 minutes using high-speed centrifugation. Then, the supernatant is collected again and centrifuged at 90,000-120,000xg for 80 to 100 minutes, followed by discarding the supernatant to obtain the exosomes remaining in the lower layer.

In an embodiment of the present disclosure, the method may further comprise a pretreatment step of pretreating induced pluripotent stem cell-derived mesenchymal stem cells with interferon gamma.

In an embodiment of the present disclosure, the method may further comprise a selective culturing step of isolating SSEA-4 (-) cells among the cultured induced pluripotent stem cells and culturing same to differentiate into BxC stem cells.

In an embodiment of the present disclosure, the method may further comprise a cell exosome production step of culturing stem cells using a cell culture medium.

The exosome production step according to the present disclosure is a process of inducing the secretion or production of exosomes from stem cells. In the present disclosure, the cell culture medium may include all media commonly used for culturing stem cells in the art. For example, commercially available media such as DMEM (Dulbecco's Modified Eagle's Medium), MEM (Minimal Essential Medium), BME (Basal Medium Eagle), RPMI 1640, DMEM/F-10 (Dulbecco's Modified Eagle's Medium: Nutrient Mixture F-10), DMEM/F-12 (Dulbecco's Modified Eagle's Medium: Nutrient Mixture F-12), α-MEM (a-Minimal Essential Medium), G-MEM (Glasgow's Minimal Essential Medium), IMDM (Iscove's Modified Dulbecco's Medium), KnockOut DMEM, and E8 (Essential 8 Medium), or artificially synthesized media may be used, but with no limitations thereto.

In an embodiment of the present disclosure, the cell culture medium may further include components such as carbon sources, nitrogen sources, trace elements, amino acids, and antibiotics.

In an embodiment of the present disclosure, the exosome production step may include an additional culturing step of culturing stem cells in exosome-depleted fetal bovine serum (FBS).

Unlike general FBS, which contains many exosomes derived from bovine serum, exosome-depleted FBS in the cell culture medium has had exosomes removed, which can prevent the incorporation of exosomes derived from FBS in addition to the exosomes secreted by the stem cells.

Another aspect of the present disclosure pertains to a method for preparing a composition comprising exosomes, the method comprising the following steps:
a first culturing step of culturing induced pluripotent stem cells (iPSCs) in a medium;
a selective culturing step of isolating SSEA-4 (-) cells among the cultured iPSCs and culturing same to differentiate into BxC stem cells;
a second culturing step of culturing the BxC stem cells to differentiate into mesenchymal stem cells;
a pretreatment step of pretreating the mesenchymal stem cells with interferon gamma;
a production step of culturing the pretreated mesenchymal stem cells to produce exosomes; and
a separation step of isolating exosomes from the mesenchymal stem cells or a culture thereof.

In an embodiment of the present disclosure, the first culturing step may involve culturing induced pluripotent stem cells in a medium containing FBS and bFGF for 1 to 10 days.

In an embodiment of the present disclosure, the selective culturing step may involve isolating SSEA-4 (-) cells among the induced pluripotent stem cells and culturing same in a medium containing FBS and bFGF for 1 to 10 days to differentiate into BxC stem cells.

In an embodiment of the present disclosure, the pretreatment step may involve culturing mesenchymal stem cells in a medium containing interferon gamma at concentrations of 0.001 to 1,000 ng, 0.005 to 500 ng, 0.01 to 100 ng, 0.05 to 50 ng, 0.1 to 25 µM, 0.5 to 15 ng, 1 to 10 µM, or 5 to 15 ng.

In an embodiment of the present disclosure, the production step may include an additional culturing step of culturing the mesenchymal stem cells in exosome-depleted fetal bovine serum (FBS).

In the present disclosure, the method may be a method for preparing a pharmaceutical composition for alleviating, inhibiting, preventing, or treating cancer, the composition comprising exosomes isolated from induced pluripotent stem cell-derived mesenchymal stem cells or a culture thereof as an active ingredient.

Another aspect of the present disclosure pertains to a method for alleviating or treating cancer, the method comprising the following step of:
administering a pharmaceutical composition comprising exosomes isolated from stem cells or a culture thereof as an active ingredient to a subject.

In an embodiment of the present disclosure, the exosomes isolated from stem cells or a culture thereof may be one or more exosomes selected from the group consisting of exosomes derived from BxC stem cells (BxC-e) and exosomes derived from BxC-I17 stem cells (BxC-I17e).

Since the treatment method according to the present disclosure includes the same exosomes isolated from stem cells or a culture thereof, as in the aforementioned composition, the common content therebetween is omitted to avoid undue complexity in this specification.

The term "treatment", as used herein, refers to any action wherein a disease is palliated or favorably altered by the administration of the composition according to the present disclosure.

The term "administration", as used herein, means providing a predetermined substance to a patient by any appropriate method. The route of administration of the pharmaceutical composition of the present disclosure may be oral or parenteral via any general route as long as it can reach the target tissue. Additionally, the composition of the present disclosure may be administered using any device capable of delivering the active ingredient to target cells.

The term "subject", as used herein, is not particularly limited but may include, for example, humans, monkeys, cows, horses, sheep, pigs, chickens, turkeys, quails, cats, dogs, mice, rats, rabbits, or guinea pigs, and may be, for example, a human, but is not limited thereto.

In an embodiment of the present disclosure, the pharmaceutical composition of the present disclosure may be administered alone but is generally administered in combination with a pharmaceutically acceptable carrier selected in consideration of the administration method and standard pharmaceutical practice.

Another aspect of the present disclosure pertains to a use of a composition including stem cell-derived exosomes as an active ingredient for alleviating, inhibiting, preventing, or treating cancer.

### Advantageous Effects of Invention

The present disclosure relates to an anti-cancer composition comprising stem cell-derived exosomes and a method for preparing same. The composition according to the present disclosure exhibits excellent anti-cancer effects and can be used as a therapeutic agent for cancer, either independently or in combination with existing anti-cancer drugs.

### Brief Description of Drawings

FIG. 1 shows graphs depicting the results of observing viability after treating the liver cancer cell lines SNU449 and Huh7 with BxC-I17e exosomes according to an embodiment of the present disclosure.
FIG. 2 is a graph depicting the results of observing viability after treating the pancreatic cancer cell line MIA-PaCa-2 with BxC-I17e exosomes according to an embodiment of the present disclosure.
FIG. 3 shows graphs depicting the results of observing viability after treating the glioblastoma cell lines HS683, U87MG, and A172 with BxC-I17e exosomes according to an embodiment of the present disclosure.
FIG. 4 shows graphs depicting the results of observing viability after treating the colon cancer cell lines HCT116 and COLO205 with BxC-I17e exosomes according to an embodiment of the present disclosure.
FIG. 5 shows graphs depicting the results of observing shows viability after treating the breast cancer cell lines T47D and MCF7 with BxC-I17e exosomes according to an embodiment of the present disclosure.
FIG. 6 is a graph depicting the results of observing viability after treating the prostate cancer cell line PC3 with BxC-I17e exosomes according to an embodiment of the present disclosure.
FIG. 7 shows graphs depicting the results of observing viability after treating the blood cancer cell lines K-562 and U266B1 with BxC-I17e exosomes according to an embodiment of the present disclosure.
FIG. 8 is a plot showing the results of observing the changes in tumor size after administering BxC-I17e exosomes to a prostate cancer xenograft tumor model according to an embodiment of the present disclosure.
FIG. 9 is a plot showing the results of observing the changes in tumor size after administering BxC-I17e exosomes to a liver cancer xenograft tumor model according to an embodiment of the present disclosure.

### Best Mode for Carrying out the Invention

A pharmaceutical composition comprising exosomes isolated from induced pluripotent stem cell (iPSC)-derived mesenchymal stem cells (MSCs) as an active ingredient for treating, preventing, alleviating, or inhibiting cancer.

### Mode for Carrying Out the Invention

The disclosure may be used for providing a better understanding of the present disclosure may be obtained from the following examples, which are set forth to illustrate, but are not to be construed to limit, the present disclosure.

### EXAMPLE 1: Culturing Induced Pluripotent Stem Cell-Derived Mesenchymal Stem Cells

Induced pluripotent stem cells (iPSCs) were cultured for 7 days in DMEM supplemented with 10% Fetal Bovine Serum (FBS) and 10 ng/ml bFGF. Then, among the cultured iPSCs, SSEA-4 (stage-specific embryonic antigen 4) negative [SSEA-4(-)] cells that do not express the SSEA-4 protein on the cell surface were isolated through FACS analysis to obtain precursor cells of mesenchymal stem cells derived from induced pluripotent stem cells. Subsequently, the isolated SSEA-4 (-) cells were further cultured by passage for 7 days in DMEM medium supplemented with 10% FBS and 10 ng/ml bFGF to produce BxC stem cells.

Thereafter, the BxC stem cells were further cultured in a culture medium containing High Glucose DMEM (Gibco, USA), 10% FBS (HyClone, USA), and 1% MEM Non-Essential Amino Acids Solution (100X, Gibco, USA) to fully differentiate into induced pluripotent stem cell-derived mesenchymal stem cells.

### EXAMPLE 2: Isolation and Characterization of Exosomes (BxC-e) Derived from Induced pluripotent Stem Cell-Derived Mesenchymal Stem Cells

### 2-1. Isolation of BxC-e Exosomes

The culture medium of induced pluripotent stem cell-derived mesenchymal stem cells, cultured in Example 1, was collected and centrifuged at 300xg for 10 minutes to remove remaining cells and cell debris. After centrifugation, the supernatant was collected, filtered using a 0.22 µm filter, and then centrifuged at 10,000xg, 4°C for 70 minutes using a high-speed centrifuge.

Subsequently, the centrifuged supernatant was collected again and centrifuged at 100,000xg, 4°C for 90 minutes using an ultracentrifuge. Then, the supernatant was removed, and the exosomes remaining in the pellet were diluted in phosphate-buffered saline (PBS) to isolate exosomes derived from induced pluripotent stem cell-derived mesenchymal stem cells (hereinafter referred to as BxC-e exosomes), which were used in the following experiments.

### 2-2. Characterization of BxC-e Exosomes

The size distribution of the BxC-e exosomes isolated in Example 2-1 was determined using nanoparticle tracking analysis (NanoSight NS300, Malvern Panalytical), and the morphology of the exosomes was observed using electron microscopy.

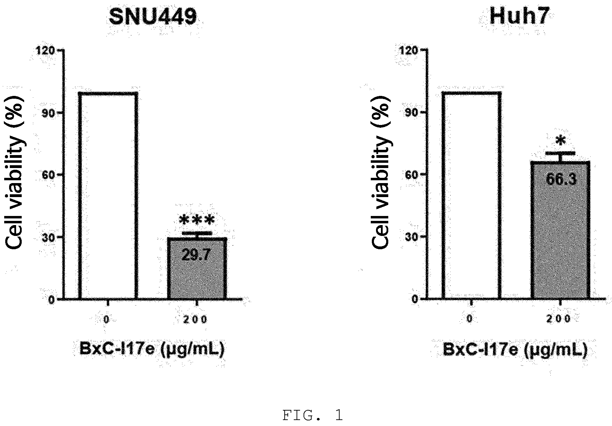

As a result, as shown in FIGS. 1a and 1b, it was confirmed that the BxC-e exosomes have the characteristics of exosomes.

### EXAMPLE 3: Isolation of Exosomes (BxC-I17e) Derived from Induced Pluripotent Stem Cell-Derived Mesenchymal Stem Cells (BxC-I17) Pretreated with Interferon Gamma

The induced pluripotent stem cell-derived mesenchymal stem cells prepared in Example 1 were cultured for 24 hours in High Glucose DMEM culture medium containing 10% Fetal Bovine Serum, 1% MEM Non-Essential Amino Acids Solution, and 20 ng/ml IFNγ to produce induced pluripotent stem cell-derived mesenchymal stem cells pretreated with IFNγ (BxC-I17 stem cells).

After completing the culture, the BxC-I17 stem cells were washed and further cultured for 72 hours in a culture medium supplemented with 10% exosome-depleted FBS.

After 72 hours of culturing, the culture medium treated with the pretreatment substance was collected and centrifuged at 300xg for 10 minutes to remove remaining cells and cell debris. Then, the supernatant was taken, filtered using a 0.22 µm filter, and centrifuged at 10,000xg, 4°C for 70 minutes using a high-speed centrifuge. Next, the centrifuged supernatant was collected again and centrifuged at 100,000xg, 4°C for 90 minutes using an ultracentrifuge to remove the supernatant, and the exosomes remaining as the pellet were diluted in PBS to isolate exosomes pretreated with IFNγ (hereinafter referred to as BxC-I17e exosomes), which were used in the following experiments.

### EXAMPLE 4: Cancer Cell Survival Assay Following Treatment with Exosomes (BxC-I17e) Derived from Interferon Gamma-Pretreated Induced Pluripotent Stem Cell-Derived Mesenchymal Stem Cells (BxC-I17)

### 4-1. Liver Cancer Cell Lines

Cells were seeded at a density of 3x10³ cells per well in a 96-well culture plate. After 16 hours, the culture medium was removed and replaced with serum-free DMEM medium containing 200 ug/ml of BxC-I17e, which was treated for 48 hours. Then, Cell Counting Kit (CCK)-8 assay solution was added to the culture medium at a 1:10 dilution. After 3 hours, the viability of the liver cancer cell lines SNU449 and Huh7 were observed and are shown in FIG. 1 and Table 1.

**TABLE 1**

| Cell line | | SNU 449 | Huh7 |
|---|---|---|---|
| Cell viability (%) | Control | 100 | 100 |
| | BxC-I17e | 29.7 | 66.3 |

As confirmed in FIG. 1 and Table 1, BxC-I17e exosomes, when administered to the liver cancer cell lines SNU449 and Huh7, significantly reduced the viability of the cancer cell line compared to the control group.

### 4-2. Pancreatic Cancer Cell Line

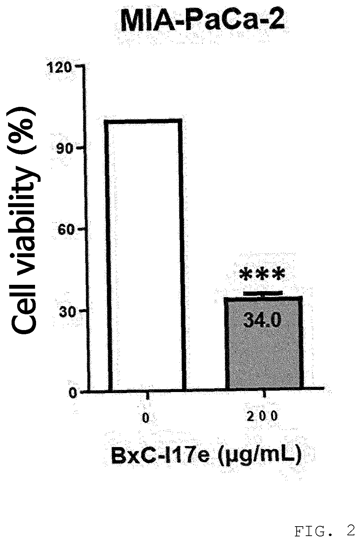

Cells were seeded at a density of 3x10³ cells per well in a 96-well culture plate. After 16 hours, the culture medium was removed and replaced with serum-free DMEM medium containing 200 ug/ml of BxC-I17e, which was treated for 48 hours. Then, Cell Counting Kit (CCK)-8 assay solution was added to the culture medium at a 1:10 dilution. After 3 hours, the viability of the pancreatic cancer cell line MIA-PaCa-2 was observed and is shown in FIG. 2 and Table 2.

**TABLE 2**

| Cell line | | MIA-PaCa-2 |
|---|---|---|
| Cell viability (%) | Control | 100 |
| | BxC-I17e | 34.0 |

As confirmed in FIG. 2 and Table 2, BxC-I17e exosomes, when administered to the pancreatic cancer cell line MIA-PaCa-2, significantly reduced the viability of the cancer cell line compared to the control group.

### 4-3. Glioblastoma Cell Lines

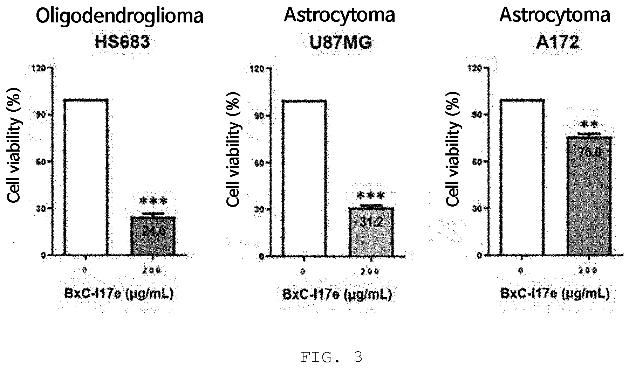

Cells were seeded at a density of 3x10³ cells per well in a 96-well culture plate. After 16 hours, the culture medium was removed and replaced with serum-free DMEM medium containing 200 ug/ml of BxC-I17e, which was treated for 48 hours. Then, Cell Counting Kit (CCK)-8 assay solution was added to the culture medium at a 1:10 dilution. After 3 hours, the viability of the glioblastoma cell lines HS683, U87MG, and A172 were observed and are shown in FIG. 3 and Table 3.

**TABLE 3**

| Cell line | | SNU 449 | Huh7 | A172 |
|---|---|---|---|---|
| Cell viability (%) | Control | 100 | 100 | 100 |
| | BxC-I17e | 24.6 | 31.2 | 76.0 |

As confirmed in FIG. 3 and Table 3, BxC-I17e exosomes, when administered to the glioblastoma cell lines HS683, U87MG, and A172, significantly reduced the viability of the cancer cell line compared to the control group.

### 4-4. Colon Cancer Cell Lines

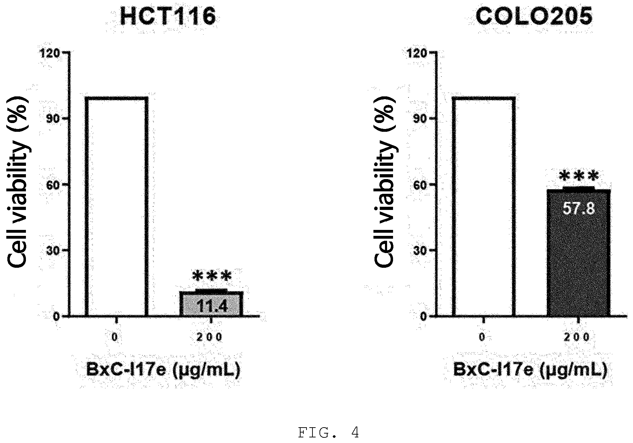

Cells were seeded at a density of 3x10³ cells per well in a 96-well culture plate. After 16 hours, the culture medium was removed and replaced with serum-free DMEM medium containing 200 ug/ml of BxC-I17e, which was treated for 48 hours. Then, Cell Counting Kit (CCK)-8 assay solution was added to the culture medium at a 1:10 dilution. After 3 hours, the viability of the colon cancer cell lines HCT116 and COLO205 were observed and are shown in FIG. 4 and Table 4.

**TABLE 4**

| Cell line | | HCT116 | COLO205 |
|---|---|---|---|
| Cell viability (%) | Control | 100 | 100 |
| | BxC-I17e | 11.4 | 57.8 |

As confirmed in FIG. 4 and Table 4, BxC-I17e exosomes, when administered to the colon cancer cell lines HCT116 and COLO205, significantly reduced the viability of the cancer cell line compared to the control group.

### 4-5. Breast Cancer Cell Lines

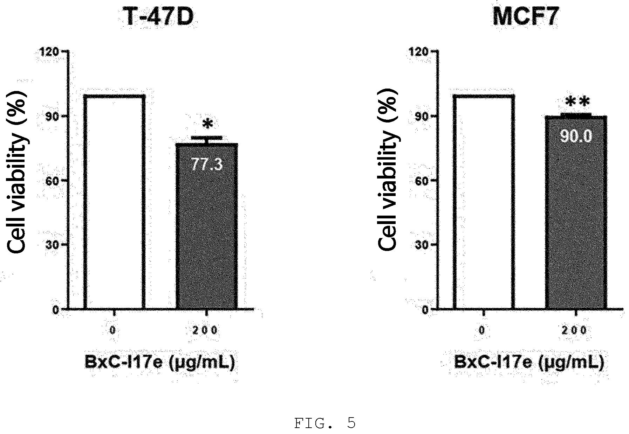

Cells were seeded at a density of 3x10³ cells per well in a 96-well culture plate. After 16 hours, the culture medium was removed and replaced with serum-free DMEM medium containing 200 ug/ml of BxC-I17e, which was treated for 48 hours. Then, Cell Counting Kit (CCK)-8 assay solution was added to the culture medium at a 1:10 dilution. After 3 hours, the viability of the breast cancer cell lines T-47D and MCF7 were observed and are shown in FIG. 5 and Table 5.

**TABLE 5**

| Cell line | | T-47D | MCF7 |
|---|---|---|---|
| Cell viability (%) | Control | 100 | 100 |
| | BxC-I17e | 77.3 | 90.0 |

As confirmed in FIG. 5 and Table 5, BxC-I17e exosomes, when administered to the breast cancer cell lines T-47D and MCF7, significantly reduced the viability of the cancer cell line compared to the control group.

### 4-6. Prostate Cancer Cell Line

Cells were seeded at a density of 3x10³ cells per well in a 96-well culture plate. After 16 hours, the culture medium was removed and replaced with serum-free DMEM medium containing 200 ug/ml of BxC-I17e, which was treated for 48 hours. Then, Cell Counting Kit (CCK)-8 assay solution was added to the culture medium at a 1:10 dilution. After 3 hours, the viability of the prostate cancer cell line PC3 was observed.

**TABLE 6**

| Cell line | | PC3 |
|---|---|---|
| Cell viability (%) | Control | 100 |
| | BxC-I17e | 38.6 |

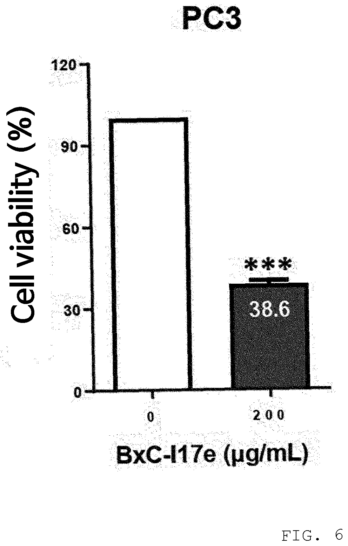

As confirmed in FIG. 6 and Table 6, BxC-I17e exosomes, when administered to the prostate cancer cell line PC3, significantly reduced the viability of the cancer cell line compared to the control group.

### 4-7. Blood Cancer Cell Lines

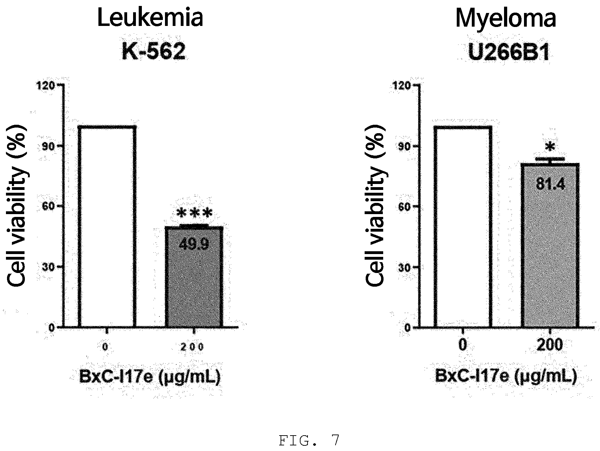

Cells were seeded at a density of 3x10³ cells per well in a 96-well culture plate. After 16 hours, the culture medium was removed and replaced with serum-free DMEM medium containing 200 ug/ml of BxC-I17e, which was treated for 48 hours. Then, Cell Counting Kit (CCK)-8 assay solution was added to the culture medium at a 1:10 dilution. After 3 hours, the viability of the blood cancer cell lines K-562 and U266B1 were observed and are shown in FIG. 7 and Table 7.

**TABLE 7**

| Cell line | | K-562 | U266B1 |
|---|---|---|---|
| Cell viability (%) | Control | 100 | 100 |
| | BxC-I17e | 49.9 | 81.4 |

As confirmed in FIG. 7 and Table 7, BxC-I17e exosomes, when administered to the blood cancer cell lines K-562 and U266B1, significantly reduced the viability of the cancer cell line compared to the control group.

### EXAMPLE 5: Confirmation of Tumor Size Reduction Effect in Xenograft Tumor Models Following Treatment with Exosomes (BxC-I17e) Derived from Interferon Gamma-Pretreated Induced Pluripotent Stem Cell-Derived Mesenchymal Stem Cells (BxC-I17)

### 5-1. Prostate Cancer Xenograft Tumor Model

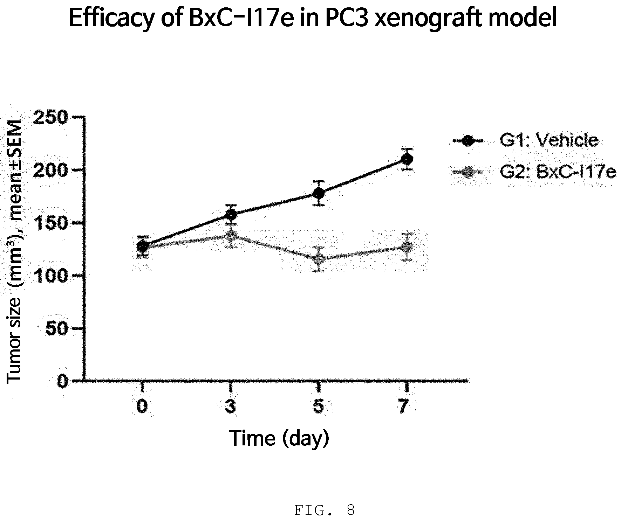

The prostate cancer cell line (PC-3) was administered to 6-week-old male SCID mice (NOD.CB17-Prkdcscid/Jcl). For cell line transplantation, the cultured cell line was checked for viability using a microscope and then prepared at a concentration of 1.0x10⁸ cells/mL. Thereafter, the dorsal area of the animal was disinfected with 70% alcohol. The skin over the dorsal area was grasped with the thumb and forefinger to create a space between the skin and muscle. A syringe fitted with a 26-gauge needle was inserted into the subcutaneous space between the thumb and forefinger from the anterior of the animal, and the cell suspension was slowly injected subcutaneously at a dose volume of 1.0x10⁷ cells/0.1 mL/head. When the tumor size at the transplantation site reached approximately 100-150 mm³, the mice were divided so that the tumor sizes in each group were as uniformly distributed as possible according to the ranked tumor sizes. BxC-I17e was then administered intratumorally using a syringe fitted with a 31-gauge needle according to each group's protocol. From the start of test substance administration (Day 0), the tumor size was observed three times a week for one week, and the results are shown in FIG. 8 and Table 8.

**TABLE 8**

| Time (day) | | 0 | 3 | 5 | 7 |
|---|---|---|---|---|---|
| Tumor Size (mm³) | Vehicle | 128.4 | 158.1 | 178.1 | 210.5 |
| | BxC-I17e | 126.7 | 137.9 | 115.7 | 127.2 |

As confirmed in FIG. 8 and Table 8, BxC-I17e exosomes were observed to effectively inhibit the growth of tumors induced by the prostate cancer cell line over time, unlike in the control group.

### 5-2. Liver Cancer Xenograft Tumor Model

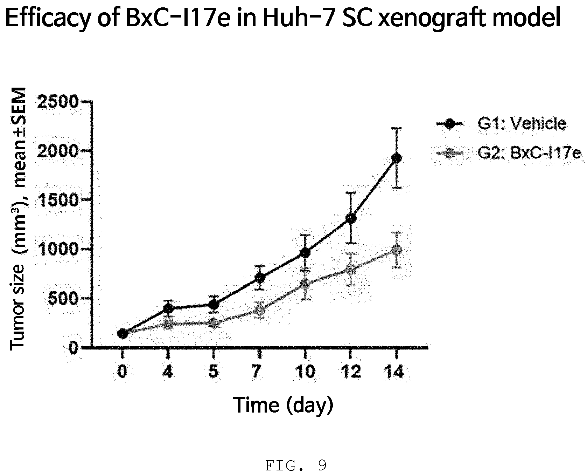

The liver cancer cell line (Huh7) was administered to 6-week-old male nude mice (CAnN.Cg-Foxn1nu/CrljOri). For cell line transplantation, the cultured cell line was checked for viability using a microscope and then cell suspension of 2.0x10⁶ cells/0.1 mL was mixed with an equal volume of Matrigel (1:1, v/v). Thereafter, the dorsal area of the animal was disinfected with 70% alcohol. The skin over the dorsal area was grasped with the thumb and forefinger to create a space between the skin and muscle. A syringe fitted with a 26-gauge needle was inserted into the subcutaneous space between the thumb and forefinger from the anterior of the animal, and the cell suspension was slowly injected subcutaneously at a dose volume of 2.0x10⁶ cells/0.2 mL/head. When the tumor size at the transplantation site reached approximately 100-150 mm³, the mice were grouped so that the tumor sizes in each group were as uniformly distributed as possible according to the ranked tumor sizes. BxC-I17e was then administered intratumorally using a syringe fitted with a 31-gauge needle according to each group's protocol. From the start of test substance administration (Day 0), the tumor size was observed three times a week for two weeks, and the results are shown in FIG. 9 and Table 9.

**TABLE 9**

| Time (day) | | 0 | 4 | 5 | 7 | 10 | 12 | 14 |
|---|---|---|---|---|---|---|---|---|
| Tumor Size (mm³) | Vehicle | 147.6 | 399.8 | 440.2 | 709.5 | 963.8 | 1317.0 | 1926.2 |
| | BxC-I17e (H) | 146.5 | 245.4 | 253.5 | 383.3 | 648.0 | 795.9 | 993.5 |

As confirmed in FIG. 9 and Table 9, BxC-I17e exosomes were observed to effectively inhibit the growth of tumors induced by the liver cancer cell line over time, unlike in the control group.

### Industrial Applicability

The present disclosure relates to a composition comprising stem cell-derived exosomes and a method for preparing the same. More specifically, the present disclosure relates to a composition comprising exosomes isolated from mesenchymal stem cells or a culture thereof, which exhibit excellent anti-cancer effects.

## Claims

1. A pharmaceutical composition comprising exosomes isolated from induced pluripotent stem cell (iPSC)-derived mesenchymal stem cells (MSCs) as an active ingredient for treating, preventing, alleviating, or inhibiting cancer.

2. The pharmaceutical composition of Claim 1, wherein the induced pluripotent stem cell-derived mesenchymal stem cells are differentiated from precursor cells of induced pluripotent stem cell-derived mesenchymal stem cells that do not express the stage-specific embryonic antigen 4 (SSEA-4) protein.

3. The pharmaceutical composition of Claim 1, wherein the induced pluripotent stem cells are human-derived induced pluripotent stem cells.

4. The pharmaceutical composition of Claim 1, wherein the induced pluripotent stem cell-derived mesenchymal stem cells are pretreated with a pretreatment substance.

5. The pharmaceutical composition of Claim 4, wherein the pretreatment substance is interferon gamma (IFN-γ).

6. The pharmaceutical composition of Claim 1, wherein the cancer is one or more selected from the group consisting of breast cancer, lung cancer, gastric cancer, liver cancer, blood cancer, bone cancer, pancreatic cancer, skin cancer, head or neck cancer, cutaneous or ocular melanoma, uterine sarcoma, ovarian cancer, rectal cancer, anal cancer, colon cancer, fallopian tube cancer, endometrial cancer, cervical cancer, small intestine cancer, endocrine cancer, thyroid cancer, parathyroid cancer, kidney cancer, soft tissue tumors, urethral cancer, prostate cancer, bronchial cancer, glioblastoma, or bone marrow cancer.

7. A food composition for palliation, inhibition, or alleviation of cancer, the composition comprising exosomes isolated from induced pluripotent stem cell (iPSC)-derived mesenchymal stem cells (MSC) as an active ingredient.

8. The food composition of Claim 7, wherein the induced pluripotent stem cell-derived mesenchymal stem cells are differentiated from precursor cells of induced pluripotent stem cell-derived mesenchymal stem cells that do not express the stage-specific embryonic antigen 4 (SSEA-4) protein.

9. The food composition of Claim 7, wherein the induced pluripotent stem cells are human-derived induced pluripotent stem cells.

10. The food composition of Claim 7, wherein the induced pluripotent stem cell-derived mesenchymal stem cells are pretreated with a pretreatment substance.

11. The food composition of Claim 10, wherein the pretreatment substance is interferon gamma (IFN-γ).

12. The food composition of Claim 7, wherein the cancer is one or more selected from the group consisting of breast cancer, lung cancer, gastric cancer, liver cancer, blood cancer, bone cancer, pancreatic cancer, skin cancer, head or neck cancer, cutaneous or ocular melanoma, uterine sarcoma, ovarian cancer, rectal cancer, anal cancer, colon cancer, fallopian tube cancer, endometrial cancer, cervical cancer, small intestine cancer, endocrine cancer, thyroid cancer, parathyroid cancer, kidney cancer, soft tissue tumors, urethral cancer, prostate cancer, bronchial cancer, glioblastoma, or bone marrow cancer.

13. A method for preparing a pharmaceutical composition for treating cancer, the method comprising the following steps:
a first culturing step of culturing induced pluripotent stem cells in a medium;
a selective culturing step of isolating SSEA-4 (-) cells among the cultured induced pluripotent stem cells and culturing same to differentiate into BxC stem cells;
a second culturing step of culturing the BxC stem cells to differentiate into mesenchymal stem cells;
a pretreatment step of pretreating the mesenchymal stem cells with interferon gamma (IFN-γ);
a production step of culturing the pretreated mesenchymal stem cells to produce exosomes; and
a separation step of isolating exosomes from the mesenchymal stem cells or a culture thereof.
